# EUROPEAN PATENT APPLICATION

(11) **EP 2 842 590 A1**
(43) Date of publication of application: **04.03.2015**
(21) Application number: 13290207.3
(22) Date of filing: 03.09.2013
(51) Int. Cl.: A61M 25/00

(54) **Access sheath**

(71) Applicant: Coloplast A/S, 3050 Humlebaek (DK)
(72) Inventor: Callede, David, 24200 Sarlat la Caneda (FR); Pascal, Laurent, 24200 Sarlat La Caneda (FR)

(57) **Abstract**

The present invention concerns an access sheath intended for positioning a tool in a working position during an intervention on a patient. Said access sheath 10 comprises a guide tube 11 and a bucket 13. The access sheath 10 of the invention is characterized in that said guide tube 11 and said bucket 13 are connected together by a connecting element 14, said connecting element enabling a rotation of the guide tube 11 relative to the bucket 13 and preventing a longitudinal movement of the guide tube 11 relative to the bucket 13.

## Description

### TECHNICAL FIELD

The present invention concerns an access sheath intended for positioning a tool in a working position during an intervention on a patient, said access sheath comprising a guide tube and a bucket.

Such an access sheath can be used on its own, in particular for inserting tools into an area of the body where an intervention has to take place. The access sheath of the invention can also be part of an assembly further comprising a dilator that may be inserted into the access sheath and a guide for guiding the access sheath during its insertion into the patient's body.

### BACKGROUND ART

When a surgeon wants to gain access to a kidney, if surgical intervention is not an option, he has to introduce an access sheath inside the urethra starting from a natural route of entry, pass the bladder and then go up inside the ureter to reach the kidney.

The positioning of such an access sheath can be performed by several methods. According to a first method, a first radio-opaque guide is driven up to the bladder by the aid of a cystoscope previously introduced inside the bladder. Then, with the aid of the cystoscope, the urethral meatus is targeted in order to insert the guide into the urethra. The guide is a generally sheathed nickel, titanium and/or stainless steel alloy lead.

After the setting up of this first guide, a radio-opaque double channel urethral probe or dilator is engaged by one of its two channels onto the guide and is driven up to the urethra. Through the other channel of the urethral probe, a second guide is inserted until it reaches the urethra. Then the urethral probe is removed, leaving behind only the two guides : a working guide and a security guide, this latter being fixed to the patient.

During these first steps, the radio-opaque components have been visualized to check their positions.

The access sheath being threaded onto a dilator projecting forwards outside the sheath, the working guide is engaged into the access sheath through the dilator channel. The access sheath is driven up to a position between the bladder and the kidney, but nearer the bladder. The dilator and the working guide are removed to leave behind in place only the access sheath, and near it, the security guide that can be used in case of difficulty.

According to a second, more efficient method, the positioning of the access sheath requires the use of only one guide and a dilator comprising at least a hole at its distal end, i.e. the end that is in the area of the intervention when the access sheath is in use. The dilator further comprises an opening connected to the hole by a channel comprising a slit on its periphery. Said opening is positioned outside of the access sheath when the access sheath and the dilator are assembled. Such an access sheath/dilator assembly is described in the patent application WO 2009/127216.

As in the previous embodiment, a guide is driven up to the bladder of the patient. The access sheath/dilator assembly is engaged with the guide, this guide entering the hole at the distal end of the dilator, following the channel and exiting the dilator through the opening.

The access sheath/dilator assembly is positioned by following the guide until it reaches the final position. The dilator is then pulled out of the access sheath. This has the effect of applying strength on the guide which in turn opens the slit of the dilator and releases the guide from the dilator. Thus, the access sheath is ready for use, with the guide following the access sheath on the external side.

Once the access sheath in position, it may happen that the bucket is not in an optimal working position, due to the rotation of the guide tube during the introduction of the access sheath within the patient's body. Subsequent rotation of the access sheath to bring the bucket into an adequate position may be difficult, impossible or may lead to injuries or pain.

The object of this invention is to provide an access sheath which can always be positioned in an optimal position, chosen by the surgeon. This optimal positioning is obtained without pain or disadvantage for the patient.

### DISCLOSURE OF INVENTION

The object of the invention is achieved by an access sheath such as defined in the preamble and **characterized in that** said guide tube and said bucket are connected together by a connecting element, said connecting element enabling a rotation of the guide tube relative to the bucket and preventing a longitudinal movement of the guide tube relative to the bucket.

According to the present invention, the access sheath can be used in cooperation with different kinds of tools such as for example an endoscope, tools for grasping objects, tools for cutting tissues, tools with laser beams... These tools are usually introduced in the access sheath once this access sheath is in place within the body of the patient, an end of said access sheath being placed in the area where the intervention takes place.

The access sheath can always be placed in a position that is optimal for the surgeon, without pain for the patient.

### BRIEF DESCRIPTION OF DRAWINGS

The present invention and its advantages will be better understood with reference to the enclosed drawing and to the detailed description of a specific embodiment, in which :
- Fig. 1 is a partial cross section view of the access sheath of the invention in a dismantled position; and
- Fig. 2 is a view similar to Fig. 1 in a set up position.

### BEST MODE FOR CARRYING OUT THE INVENTION

With reference to the figures, the access sheath 10 of the present invention is intended for receiving a tool (not represented) that will be used in an area of a body which is difficult to access. This tool can be an endoscope or another device for taking pictures of the inner part of the patient's body, tools for grasping objects, tools for cutting patient's tissues, tools for delivering drugs, liquids or other products or tools comprising laser beams for example. The access sheath 10 comprises a guide tube 11 comprising a longitudinal hole 12 for receiving and guiding the tool. The access sheath further comprises a bucket 13 having a general shape of a funnel with a hole coinciding with the hole 12 of the guide tube 11.

The access sheath 10 of the invention further comprises a connecting element 14, intended for connecting the guide tube 11 to the bucket 13, in such a way that the guide tube can rotate with regard to the bucket. Moreover, the connecting element 14 is conformed to prevent the bucket 13 and the guide tube 11 from moving in a longitudinal direction with regard to each other.

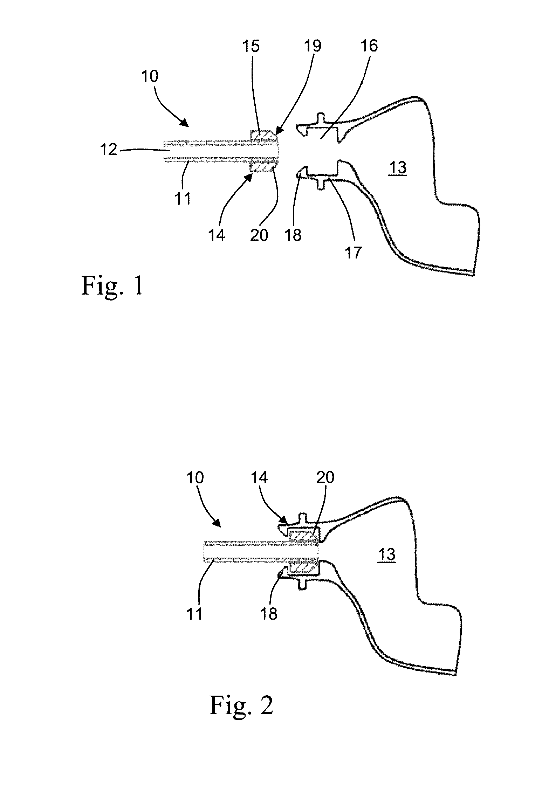

According to a preferred embodiment, illustrated by Fig. 1 and 2, the connecting element 14 comprises a ring 15 integral with the guide tube 11 and positioned at a proximal end of this guide tube. This ring 15 cooperates with an annular housing 16 formed in the bucket 13. This annular housing 16 has a dimension allowing for the introduction of the ring 15 and for the rotation of the ring in the housing. This annular housing 16 is delimited by elastic walls 17 and by a retaining area 18 on the front of the housing, i.e. in the direction of the guide tube.

The ring 15 of the connecting element 14 further comprises a means 19 for moving the retaining area, said means comprising advantageously an inclined plane 20.

Fig. 1 illustrates the access sheath of the invention in which the guide tube 11 and the bucket 13 are separated. Fig. 2 illustrates the guide tube 11 with the bucket 13 and the guide tube mounted and ready for use.

When the guide tube 11 and the bucket 13 have to be mounted, the guide tube 11 is pushed against the bucket 13, the inclined planes 20 of the means 19 for moving the retaining area being pushed against this retaining area 18. As the walls of the annular housing 16 are elastic, the pressure of the ring 15 against the retaining area 18 deforms the elastic walls and opens the retaining area. The ring 15 can then enter the annular housing 16, as illustrated by Fig. 2

The retaining area 18 prevents the ring 15 from exiting the annular housing 16. Due to the respective dimensions of the ring 15, the annular housing 16 and the retaining area 18, the guide tube 11 and the bucket 13 can rotate with respect to each other. However, the guide tube 11 and the bucket 13 cannot move in a longitudinal direction with respect to each other.

In the embodiment illustrated, the distal end of the ring 15 does not comprise means for moving the retaining area. Accordingly, the retaining means prevents the guide tube 11 from being separated from the bucket 13 simply by pulling these elements apart. However, it is possible to provide the ring 15 with such means, for example having the shape of inclined planes, in order to enable the guide tube 11 and the bucket 13 to be separated by pulling them apart. If such means for moving the retaining area are not provided, the guide tube 11 and the bucket 13 could be separated for example by using a tool enabling the opening of the retaining area.

The present invention enables the rotation of the bucket with regard to the guide tube. It further enables the replacement or the selection of a bucket. Hence, in some cases, it could be interesting to use a particular bucket having for example a specific shape enabling the use of specific functions. As the guide tube and the bucket are two separate elements, it is possible to choose one guide tube among a specific assortment and to connect said guide tube to a selected bucket among an assortment of buckets.

## Claims

1. Access sheath intended for positioning a tool in a working position during an intervention on a patient, said access sheath (10) comprising a guide tube (11) and a bucket (13), **characterized in that** said guide tube (11) and said bucket (13) are connected together by a connecting element (14), said connecting element enabling a rotation of the guide tube (11) relative to the bucket (13) and preventing a longitudinal movement of the guide tube (11) relative to the bucket (13).

2. Access sheath according to claim 1, **characterized in that** the connecting element (14) comprises a ring (15) integral with the guide tube (11) and **in that** said bucket (13) comprises an annular housing (16) for receiving said ring (15).

3. Access sheath according to claim 2, **characterized in that** said annular housing (16) comprises a retaining area (18) for preventing the ring (15) from exiting the annular housing (16).

4. Access sheath according to claim 2, **characterized in that** the annular housing (16) has elastic walls (17) and **in that** said retaining area (18) is mobile through the deformation of the elastic walls (17).

5. Access sheath according to claim 4, **characterized in that** the ring (15) comprises a means (19) for moving the retaining area (18).

6. Access sheath according to claim 5, **characterized in that** said means (19) for moving the retaining area comprises an inclined plane (20).
